(19) **Europäisches Patentamt / European Patent Office / Office européen des brevets**

(11) **EP 4 442 697 A1**

(12) **EUROPEAN PATENT APPLICATION**

(43) Date of publication:
**09.10.2024 Bulletin 2024/41**

(21) Application number: **23167140.5**

(22) Date of filing: **06.04.2023**

(51) International Patent Classification (IPC):
**C07K 14/00** (2006.01) **C07K 14/78** (2006.01)
**C12N 15/81** (2006.01) **C12P 21/02** (2006.01)
**C12R 1/84** (2006.01)

(52) Cooperative Patent Classification (CPC):
**C07K 14/78; C07K 14/00; C12N 15/815;
C12P 21/02;** C12R 2001/84

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
- **Stichting Wageningen Research
  6708 PB Wageningen (NL)**
- **Tate & Lyle Solutions USA LLC
  Hoffman Estates, IL 60192 (US)**

(72) Inventors:
- **WERTEN, Marc, Willem, Theodoor
  6708 PB Wageningen (NL)**
- **YOU, Zheng
  Hoffmann Estates, IL 60192 (US)**

(74) Representative: **V.O.
P.O. Box 87930
2508 DH Den Haag (NL)**

Remarks:
Claims 16-23 are deemed to be abandoned due to non-payment of the claims fees (Rule 45(3) EPC).

(54) **GELATIN-REPLACEMENT POLYPEPTIDES**

(57) Disclosed is a gelatin-replacement polypeptide that can be suitably produced in *Pichia pastoris.* The polypeptide has a structure $(T_{block}\text{-}G_{block})_n\text{-}T_{block}$, wherein n is an integer of from 1 to 18. $T_{block}$ is, for example, $(GPP)_k$, with k being 6-20. $G_{block}$ comprises the oligopeptides having the amino acid sequences GPKGEPGSPGEN, GPKGNSGEP, GPSGEPGKQGPS, and GSPGEQ, preferably $G_{block}$ has the amino acid sequence $(GPKGEPGSPGENGPKGNSGEPGPSGEPGKQGPSGSPGEQ)_q$ with q being 1 to 40.

**EP 4 442 697 A1**

## Description

### Field

The invention is in the field of gelatin-replacement polypeptides. Particularly, the invention pertains to tri- and multi-block polypeptides capable of being produced in yeast host cells, and capable of gelling.

### Background

Natural gelatin is regularly obtained by heat denaturation, i.e. a disintegration of the triple helix structure, after hydrolysis of the intermolecular covalent crosslinks in fibrillar collagen from animal sources, notably type I and type III collagen from bovine or porcine origin. Collagen is made up of three polypeptide strands. Each of these is a left-handed extended helix, and together they are twisted into a triple helix. Animal collagen is held together by intermolecular crosslinks that are formed after formation of the triple helical collagen molecules themselves and their organization into fibrils, the degree of crosslinking increasing with the age of the tissue. These crosslinks must be largely hydrolyzed in order to make the collagen and - after heat denaturation - the resulting gelatin, soluble. However, the necessary hydrolysis, brought about at extreme pH, also results in unwanted and largely uncontrollable partial hydrolysis of the collagen backbone, and (when treated at high pH) in deamidation and shifting of the isoelectric point.

As a further drawback of natural gelatin, *inter alia* the animal origin is seen as a risk, in view of the chances that the animal collagen may carry transmissible disease agents, e.g. spongiform encephalopathy prions, such as the well-known bovine spongiform encephalopathy (BSE), which is seriously suspected to be linked to the fatal neurological disease of Creutzfeldt-Jakob in humans. In food, and even more strongly in pharmaceutical applications, this is a critical issue, which has resulted in regulatory restrictions on the origin of the gelatins used in these products. Moreover, a tendency in society is to try and reduce cattle breeding in view of environmental concerns. As a result, an emerging desire is to replace proteins traditionally derived from animal sources, by proteins produced in an animal-free manner, yet with the same functionalities. Gelatin is an outstanding example of proteins for which it is sought to meet this desire. The foregoing comes in addition to previously existing reasons to avoid animal-produced proteins, such as reasons based on animal welfare (vegetarian or vegan life style) or religious reasons. Interestingly, Yeast-derived, fungal or bacterial products can be considered to be suitable replacements as being, *inter alia,* vegan as well as kosher or halal.

Thus, it is desirable to be able to avoid animal collagen as a source for gelatin. To this end WO 2009/151327 proposes a block co-polypeptide comprising at least two trimerizing blocks and at least one spacer block. The disclosure emphasizes the tunability of the polypeptide structures to be produced, and represents that the block co-polypeptide is capable of forming a gel, and is useful as an animal-free, biocompatible substitute for gelatin. Background art related to these triblock designed polypeptides is Werten et al., Biomacromolecules (2009), Vol.10, pp 1106-1113. Further background literature on the production of custom-designed gelatins in *Pichia pastoris* is Werten et al., Protein Engineering (2001), vol.14, no.6, pp 447-454. All of these references are directed at avoiding animal polypeptide sources, and advocating custom-designed gelatins.

Producing artificial, mammalian (e.g. bovine or porcine), designed polypeptide, also comes with a disadvantage. On the one hand, it is desired to avoid animal gelatin. On the other hand, the public at large prefers natural products over artificial products, particularly with respect to food. In view hereof, it is preferred to step away from fully artificial peptide sequences, and build a polypeptide made up of stretches of natural sequences.

The foregoing artificial polypeptides are produced as extracellular proteins in recombinant yeast, specifically in transformed *Pichia pastoris.* Background art on such production in *Pichia pastoris* can be found in Werten et al., Yeast 15(11):1087-1096. In view of, *inter alia,* its GRAS status for heterologous protein production, *Pichia pastoris* provides a desirable platform for the production of recombinant gelatins. Nonetheless, it is quite challenging to find natural gelatin-like sequences that actually can be produced as intact proteins in *Pichia pastoris.* This relates to the phenomenon that many polypeptide sequences will be susceptible to proteolytic cleavage by protein processing enzymes naturally present in *Pichia pastoris.* This is particularly true for unfolded proteins such as gelatins. Also the gel forming ability of gelatin-like polypeptides produced in *Pichia* is not normally achieved. Natural collagen sequences, when produced by *Pichia,* do not have the aforementioned triple helix-forming and gel-forming capacity. This is because, in contrast to animal collagen-producing cells, *Pichia* does not naturally carry out the post-translational modifications required for this capacity.

It is thus desired to provide a gel-forming polypeptide suitable as a gelatin replacement, that can be produced in an animal-free manner, preferably in *Pichia pastoris,* yet comprising, preferably consisting of natural peptide sequences.

### Summary of the invention

In order to better address one or more of the above desires, the invention presents, in one aspect, a polypeptide having an amino acid sequence comprising the oligopeptides of formula (II), (III), (IV), and (V):

GPKGEPGSPGEN (II)

GPKGNSGEP (III)

GPSGEPGKQGPS (IV)

GSPGEQ (V),

preferably a polypeptide having the sequence of formula (I)

(GPKGEPGSPGENGPKGNSGEPGPSGEPGKQGPSGSPGEQ)q (I)

wherein q is an integer of from 1 to 40.

In another aspect, the invention resides in a block co-polypeptide having a structure $(T_{block}\text{-}G_{block})_n\text{-}T_{block}$, wherein:

- n is an integer of from 1 to 18;
- $T_{block}$, each independently denotes a trimerizing peptide block selected from the group consisting of $(GPP)_k$, $(GER)_m$, $(GRE)_m$, $(GEK)_m$, $(GKE)_m$, $(GPKGDP)_p$, and $(GPKGEP)_p$, wherein k is an integer from 6 to 20, preferably 7 to 16, more preferably 8 to 12, m is an integer of from 12 to 20 and p is an integer of from 3 to 12;
- $G_{block}$ represents a peptide block satisfying the structure

(GPKGEPGSPGENGPKGNSGEPGPSGEPGKQGPSGSPGEQ)q,

wherein q is an integer of from 1 to 40;

the capital letter designations defining the peptide blocks in accordance with the conventional one-letter nomenclature for amino acids.

In a further aspect, the invention provides a hydrogel comprising a block co-polypeptide as defined in the preceding paragraph.

In still further aspects, the invention resides in a nucleic acid molecule encoding any one the polypeptide and block polypeptide sequences as defined in the preceding paragraphs; an expression vector comprising said nucleic acid molecule; and a host cell comprising said nucleic acid molecule.

In yet another aspect, the invention provides a method of preparing a polypeptide, comprising:

a) introducing the aforementioned expression vector into a host cell;
b) culturing the host cells in a culture medium, under conditions allowing the expression of the polypeptide in said host cells and secretion of the polypeptide into the culture medium;
c) removing said host cells from the culture medium, so as to provide a cell-free culture medium containing the secreted polypeptide by microfiltration, optionally preceded by centrifugation;
d) optionally concentrating the polypeptide in the cell-free culture medium by ultrafiltration
e) optionally removing low molecular weight components from the polypeptide and culture medium by ultrafiltration/diafiltration
f) precipitating specifically the polypeptide from the cell-free culture medium by differential precipitation with a salt, preferably with ammonium sulfate, preferably at 20-80% of the saturating ammonium sulfate concentration, more preferably at 40-60% of the saturating ammonium sulfate concentration;
g) dissolving the precipitated polypeptide in water;
h) optionally repeating the above precipitation and dissolution of the polypeptide once, to increase the purity of the polypeptide;
i) removing the salt from the polypeptide and optionally concentrating the polypeptide by ultrafiltration/diafiltration or dialysis,
j) drying the polypeptide by spray drying or freeze-drying.

In yet another aspect, the invention provides a method of preparing a polypeptide comprising:

a) introducing the aforementioned expression vector into a host cell;
b) culturing the host cells in a culture medium, under conditions allowing the expression of the polypeptide in said host cells and secretion of the polypeptide into the extracellular culture medium;

c) removing said host cells from the culture medium, so as to provide a cell-free culture medium containing the secreted polypeptide by microfiltration, optionally preceded by centrifugation;

d) optionally concentrating the polypeptide in the cell-free culture medium by ultrafiltration

e) optionally removing low molecular weight components from the polypeptide and culture medium by ultrafiltration/diafiltration

f) purifying the polypeptide by chromatography, for example anion exchange chromatography

g) removing salts and/or buffers from the polypeptide and optionally concentrating the polypeptide by ultrafiltration/diafiltration or dialysis,

h) drying the polypeptide by spray drying or freeze-drying.

In yet another aspect, the invention provides a method of preparing a polypeptide, comprising:

a) introducing the expression vector of claim 7 into host cells;

b) culturing the host cells in a culture medium, under conditions allowing the expression of the polypeptide in said host cells;

c) lysing the host cells resulting in a dispersion comprising the polypeptide and lysed cell debris;

d) separating said debris from the dispersion, by a separation technique selected from the group consisting of centrifugation,, ultrafiltration, microfiltration, and combinations thereof;

e) optionally removing low-molecular weight components from the polypeptide-containing dispersion by ultrafiltration or dialysis;

f) purifying the polypeptide by a combination of differential precipitation and or chromatography;

g) desalting the polypeptide obtained in step (d), (e) or (f) by ultrafiltration or dialysis and optionally drying the polypeptide.

## Drawings

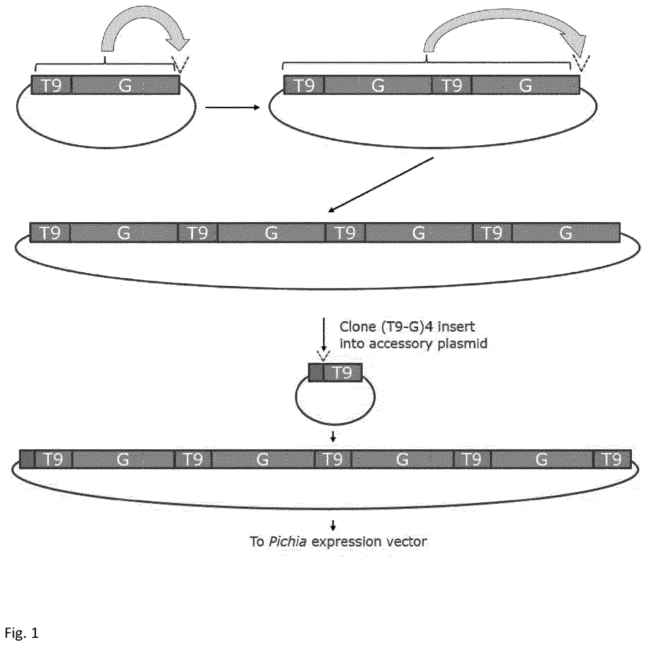

Fig. 1 schematically shows a cloning procedure to generate a gene construct encoding a block co-polypeptide of the invention.

## Detailed description

The invention generally relates to gellable polypeptides comprising at least two trimerizing blocks ("$T_{block}$') and at least one not intrinsically trimerizing block ("$G_{block}$'), the alternating $T_{block}$ and $G_{block}$ modules in the polypeptide providing gelling capability. In a broad sense, the invention is based on the judicious insight to thereby provide, for the $G_{block}$ a novel polypeptide sequence consisting of a combination of natural polypeptide stretches found in different bovine type I alpha1 collagen sequences. Said novel polypeptide sequence is described, according to the one letter nomenclature for polypeptides as having the amino acid sequence according to Formula (I):

$$(GPKGEPGSPGENGPKGNSGEPGPSGEPGKQGPSGSPGEQ)q \qquad (I)$$

wherein q is an integer of from 1 to 40. Preferably q is 2 to 20, more preferably 3 to 10. Still more preferably q is 3-8, most preferably 5.

In amino acid sequences, as defined herein, amino acids are denoted by single-letter symbols. These single-letter symbols and three-letter symbols are well known to the person skilled in the art. For completeness' sake, we note that for the amino acids referred to in the present disclosure the single-letter symbols have the following meaning: A (Ala) is alanine, D (Asp) is aspartic acid, E (Glu) is glutamic acid, G (Gly) is glycine, K (Lys) is lysine, N (Asn) is asparagine, P (Pro) is proline, Q (Gln) is glutamine, R (Arg) is arginine, and S (Ser) is serine.

The novel polypeptide of formula (I) combines a set of four natural oligopeptide stretches, each found in bovine type I alpha1 collagen:

GPKGEPGSPGEN          (II)

GPKGNSGEP          (III)

GPSGEPGKQGPS          (IV)

GSPGEQ          (V)

Optionally, the aforementioned polypeptide of formula (I) can be produced as such. In that event, an intermediate is provided that can be used in producing a gellable polypeptide by chemically attaching trimerizing blocks to it. Although this is technically possible, those skilled in the art will appreciate that producing the entire polypeptide by means of recombinant technologies is preferred. This better allows to produce long and monodisperse polypeptides with a defined sequence order.

Polypeptide manufacture through recombinant technologies is known. In general an artificial DNA sequence is provided in a known manner, having a sequence encoding for the desired polypeptide. Various cloning methods are nowadays available for the construction of G-C-rich, repetitive DNA sequences and genes of interest (Padgett K.A. & Sorge J.A. (1996) Gene 168: 31-35; McMillan R.A., Lee T.A.T. & Conticello V.P. (1999) Macromolecules 32: 3643-3648; Werten M.W.T., Wisselink W.H., Jansen-van den Bosch T.J., de Bruin E.C. & de Wolf F.A. (2001) Protein Engineering 14(6): 447-454; Goeden-Wood N.L., Conticello V.P., Muller S.J. & Keasling J.D. (2002) Biomacromolecules 3: 874-879; Won J.-I. & Barron A.E. (2002) Macromolecules 35: 8281-8287; Henderson D.B., Davis R.M., Ducker W.A., Van Cott K.E. (2005) Biomacromolecules 6: 1912-1920; Lu Q. (2005) Trends in Biotechnol. 23(4): 199-207; Mi L. (2006) Biomacromolecules 7: 2099-2107). This does not require elucidation here. The DNA, in a suitable vector, is expressed in a host cell. Suitable hosts are e.g. *Pichia pastoris, Hansenula polymorpha, Kluyveromyces marxianus* var. *Lactis, Aspergillus nigerl oryzae / sojae / awamori, Bacillus megaterium / brevis / subtilis / thuringiensis, E. coli K12 derivative.* The preferred host is *Pichia pastoris* and the preferred mode of expression is secretion into the extracellular medium.

Thus, preferably, the polypeptide of formula (I) is produced in the form of one or more blocks contained in an overall gellable polypeptide structure. It will be understood that, in such event, the terminal amino acids of the polypeptide of formula (I) will have a peptide bond linkage to an adjacent trimerizing block. Preferably, this is accomplished in such a way as to provide a polypeptide satisfying formula

$$(T_{block}\text{-}G_{block})_n\text{-}T_{block} \qquad (VI)$$

wherein:

- n is an integer of from 1 to 18;
- $T_{block}$, each independently denotes a trimerizing peptide block selected from the group consisting of $(GPP)_k$, $(GER)_m$, $(GRE)_m$, $(GEK)_m$, $(GKE)_m$, $(GPKGDP)_p$, and $(GPKGEP)_p$, wherein k is an integer from 6 to 20, preferably 7 to 16, more preferably 8 to 12 m is an integer of from 12 to 20 and p is an integer of from 3 to 12;
- $G_{block}$ represents the aforementioned peptide block of formula (I) satisfying the structure

(GPKGEPGSPGENGPKGNSGEPGPSGEPGKQGPSGSPGEQ)q,

wherein q is an integer of from 1 to 40, preferably 2 to 20, more preferably 3 to 10, still more preferably 3-8, and most preferably 5.

In a preferred embodiment, $T_{block}$ has the peptide sequence $(GPP)_k$, with k preferably being 7 to 16, more preferably 8 to 12.

The invention further encompasses nucleic acid constructs, including DNA and RNA molecules, that encode the polypeptides described herein. Accordingly, in one embodiment, triblock $[(T_{block}\text{-}G_{block})_n\text{-}T_{block}]$ genes are constructed and transfected to an expression host, preferably P. *pastoris,* using a *Pichia pastoris* expression vector.

The invention also features vectors that include the present nucleic acid constructs. Of particular benefit are expression vectors, especially those for expression in eukaryotic cells. Such vectors can, for example, be viral, plasmid, cosmid, or artificial chromosome (e.g., yeast artificial chromosome) vectors. Typically, plasmids are circular, dsDNA elements that include one or more cloning sites for insertion of selected DNA sequences, e.g., coding sequences. Such plasmids may include a functional origin of replication and thus are replication competent, or may be replication defective. In view of the preferred production in yeast, particularly in *Pichia pastoris,* it will be understood that the corresponding yeast expression vectors are preferred, more preferably *Pichia pastoris* expression vectors, even more preferring *Pichia pastoris* expression vectors with a suitable signal sequence for secretory expression, most preferably *Pichia pastoris* expression vectors with a suitable pre-pro-sequence for efficient polypeptide secretion, and conferring stable integration into the genome of *Pichia pastoris* at a specifically targeted locus, after linearization of the expression vector with a suitable corresponding endonuclease and transfection of the linearized vector into the *Pichia* cells, for example by electroporation.

With reference to the possible repetition of the various polypeptide sequences, i.e., the aforementioned integers k, n, m, and p, corresponding cloning procedures can be carried out. Such procedures as such are well-known in the art. Reference is also made to Figure 1, which shows a cloning procedure to generate the gene constructs encoding $(T_{block}-G_{block})_n-T_{block}$ as well as the subsequent transformation of *P. pastoris.*

The present nucleic acid constructs can be introduced into the host cells growing in culture in vitro by conventional transfection techniques (e.g., calcium phosphate precipitation, DEAE-dextran transfection, electroporation, and other methods, preferably electroporation).

Another aspect of the invention pertains to host cells, preferably P. *pastoris cells,* into which a nucleic acid construct of the invention has been introduced, i.e., a "recombinant host cell", preferably with a nucleic acid construct of the invention stably integrated into the genome of *Pichia pastoris* at a specifically targeted locus

Overall, polypeptides are thus produced in a method comprising:

a) introducing an applicable expression vector, as described hereinbefore, into a host cell;
b) culturing the host cells in a culture medium, under conditions allowing the expression of the polypeptide in said host cells;
c) removing said host cells from the culture medium containing the secreted polypeptide by microfiltration, optionally preceded by centrifugation;
d) optionally concentrating the polypeptide in the cell-free culture medium by ultrafiltration
e) optionally removing low molecular weight components from the polypeptide and culture medium by ultrafiltration/diafiltration
f) precipitating specifically the polypeptide from the cell-free culture medium by differential precipitation with a salt, preferably with ammonium sulfate, preferably at 20-80% of the saturating ammonium sulfate concentration, more preferably at 40-60% of the saturating ammonium sulfate concentration;
g) dissolving the precipitated polypeptide in water;
h) optionally repeating the above precipitation and dissolution of the polypeptide once, if needed to increase the purity of the polypeptide;
i) removing the salts from the polypeptide and optionally concentrating the polypeptide by ultrafiltration/diafiltration or dialysis,
j) drying the polypeptide by spray drying or freeze-drying.

In yet another aspect, the invention provides a method of preparing a polypeptide comprising:

a) introducing the aforementioned expression vector into a host cell;
b) culturing the host cells in a culture medium, under conditions allowing the expression of the polypeptide in said host cells and secretion of the polypeptide into the extracellular culture medium;

removing said host cells from the culture medium, so as to provide a cell-free culture medium containing the secreted polypeptide by microfiltration, optionally preceded by centrifugation;

d) optionally concentrating the polypeptide in the cell-free culture medium by ultrafiltration
e) optionally removing low molecular weight components from the polypeptide and culture medium by ultrafiltration/diafiltration
f) purifying the polypeptide by chromatography, for example anion exchange chromatography
g) removing the salts from the polypeptide and optionally concentrating the polypeptide by ultrafiltration/diafiltration or dialysis,
h) drying the polypeptide by spray drying or freeze-drying.

In yet another aspect, the invention provides a method of preparing a polypeptide, comprising:

a) introducing the aforementioned expression vector into a host cell;
b) culturing the host cells in a culture medium, under conditions allowing the expression of the polypeptide in said host cells;
c) lysing the host cells;
d) separating the lysed cells,
e) solubilizing the lysed cells to provide a solution,
f) dialyzing the solution obtained in step (e), thereby obtaining isolated polypeptides
g) drying the polypeptide.

The block co-polypeptide of the invention will generally be obtained in the form of a lyophilized or spray dried polypeptide.

Just as natural gelatin, the block co-polypeptide of the invention is capable of reversibly forming hydrogels upon cooling heated aqueous solutions of the polypeptide. The temperature at which a gel is formed can be tuned by varying the length of the triple-helix forming blocks, i.e., the integers k, m., and p referred to above. Generally this temperature will vary from 10 to 80 °C, preferably from 15 to 50 °C, more preferably from 30-40 °C.

The block co-polypeptide of the invention presented in this disclosure is suitable as a substitute for gelatin in foods and beverages. Accordingly, an aspect of the invention includes the use of said block-co-polypeptide as an ingredient capable of gelling in food and beverage applications. Particularly this concerns desserts (more particularly for gel formation, and/or for texture, transparency, and brilliance), fruit gummies (more particularly for gel formation and/or for texture, elasticity, transparency and/or brilliance), marshmallows (more particularly for foam formation, and/or for foam stabilization or gel formation), pastilles (more particularly as a binding agent and/or for texture or melting properties, and to prevent disintegration), caramels (more particularly as an emulsifier and foam stabilizer, and/or for chewability), yogurt (more particularly for stabilization of syneresis, and/or for texture and creaminess), meat and sausages (more particularly for emulsion stabilization and/or water/juice binding), and broths and canned meats (more particularly as a binding agent, and/or for texture as well as sliceability of canned meats).

The block co-polypeptides of the present invention serve to better address the desire for providing an animal-free gelatin replacement, satisfying desired gelatin properties enabling its use in food applications. In addition to the foregoing, this refers to functional properties such as: clarity, elastic texture, melt in mouth, for ready to eat (RTE) dessert gels; elastic texture, clarity, low hot viscosity, low set temperature in high solids confectionery; as a whipping/aeration agent, foam stabilizer, elastic texture in foamed confectionery - marshmallows; for elastic gel texture, fatlike mouthfeel, emulsion stabilization in low-fat spreads; for a creamy mouthfeel in stirred yogurt, and prevention of syneresis; as a whipping agent in desserts and mousses, and also for a creamy consistency and providing a low set temperature; for providing a smooth texture and a creamy mouthfeel to sour cream; and for improving and stabilizing the soft texture of ice cream.

In interesting further embodiments, the block co-polypeptides of the present invention are suitable for use in various cosmetic or medical application. These include a use as:

- a rheology modifier in cosmetics or personal care formulations;
- a skin-restoring agent in cosmetics or personal care formulations;
- a material for the production of capsules for pharmaceutical formulations, i.e., capsules for the administration of substances, such as drug substances;
- a material for the production of gels for the controlled and/or extended release of substances, such as drug substances;
- a scaffold material for tissue culture or tissue engineering;
- an additive to promote the stability, viability and/or shelf life of cells, tissues, vaccines.

In these applications, the polypeptide will be generally used in the form of a hydrogel. The polypeptides of the invention can also be used, either in gel or in dry form, as a surgical aid to prevent post-operative sticking of tissues or organs to each other. Another such use, either in gel or in dry form as an aid in surgery to control blood flow, such as temporary control of blood flow during or after surgery.

In sum, a gelatin-replacement polypeptide is disclosed that can be suitably produced in *Pichia pastoris.* The polypeptide has a structure $(T_{block}\text{-}G_{block})_n\text{-}T_{block}$, wherein n is an integer of from 1 to 18. $G_{block}$ comprises the oligopeptides having the amino acid sequences GPKGEPGSPGEN, GPKGNSGEP, GPSGEPGKQGPS, and GSPGEQ. Preferably $G_{block}$ has the amino acid sequence

(GPKGEPGSPGENGPKGNSGEPGPSGEPGKQGPSGSPGEQ)q

wherein q is an integer of from 1 to 40.

The invention will be illustrated hereinafter with reference to the following non-limiting Examples.

**Example 1**

Three gelatin designs were selected, each of a general structure $(T_{block}\text{-}G_{block})_n\text{-}T_{block}$ with n = 1.

(A) $G_{block}$ consisting of a stretch of the the natural bovine collagen type I (alpha 1) sequence.
(B) $G_{block}$ consisting of a stretch of the the natural bovine collagen type III (alpha 1) sequence.
(C) $G_{block}$ consisting of the polypeptide of Formula (I).

In all three polypeptides the N- and C-terminal ends of each $G_{block}$ block were fused with a $T_{block}$ consisting of a nonablock triplet oligopeptide having the sequence (GPP)$_9$.

(A)

- 255 amino acids
- molecular weight: 22.52 kDa
- IEP: 8.34
- Amino acid sequence:

**(GPP)$_9$**-GPSGEPGKQGPSGASGERGPPGPMGPPGLAGPPGESGREGAPG AEGSPGRDGSPGAKGDRGETGPAGPPGAPGAPGAPGPVGPAGKSGDRG ETGPAGPAGPIGPVGARGPAGPQGPRGDKGETGEQGDRGIKGHRGFSGL QGPPGPPGSPGEQGPSGASGPAGPRGPPGSAGSPGKDGLNGLPGPIGPP GPRGRTGDAGPA-**(GPP)$_9$**

(B)

- 249 amino acids
- molecular weight: 21.59 kDa
- IEP: 5.20
- Amino acid sequence:

**(GPP)$_9$**-GERGGPGGPGPQGPAGKNGETGPQGPPGPTGPSGDKGDTGPPG PQGLQGLPGTSGPPGENGKPGEPGPKGEAGAPGIPGGKGDSGAPGERG PPGAGGPPGPRGGAGPPGPEGGKGAAGPPGPPGSAGTPGLQGMPGERG GPGGPGPKGDKGEPGSSGVDGAPGKDGPRGPTGPIGPPGPAGQPGDKG ESGAPGVP-**(GPP)$_9$**

(C)

- 249 amino acids
- molecular weight: 22.37 kDa
- IEP: 4.63
- Amino acid sequence:

**(GPP)$_9$**-GPKGEPGSPGENGPKGNSGEPGPSGEPGKQGPSGSPGEQGPKG EPGSPGENGPKGNSGEPGPSGEPGKQGPSGSPGEQGPKGEPGSPGENG PKGNSGEPGPSGEPGKQGPSGSPGEQGPKGEPGSPGENGPKGNSGEPG PSGEPGKQGPSGSPGEQGPKGEPGSPGENGPKGNSGEPGPSGEPGKQG PSGSPGEQ-**(GPP)$_9$**

**Example 2**

Three triblock gelatin-encoding genes, corresponding to (A), (B), and (C) of Example 1 were constructed and cloned into a *Pichia pastoris* vector. The three vectors were used to transform *Pichia.* Subsequently, benchtop methanol fed-batch fermentations were performed with selected *Pichia* strains. After cell removal by centrifugation and microfiltration, the proteins were purified from the cell-free broth by differential ammonium sulfate precipitation, and they were desalted by dialysis. For analytical purposes such as SDS-PAGE and mass spectrometry, the purification is normally done at (sub)-mL scale.

The purified proteins were subjected to polyacrylamide gel electrophoresis after treatment with SDS to denature the proteins and provide them with negative charges (SDS-PAGE). Therein polypeptide (C) showed only very minor degradation (note that the gel is overloaded). Polypeptides (A) and (B) showed some degradation, although still much less than typically seen for natural collagen sequences.

The three triblock proteins were subjected to MALDI-TOF to analyze their molecular mass distribution. This confirmed the conclusion from SDS-PAGE that proteolytic degradation is negligible in (C), while some level of degradation is visible in (A) and particularly in (B). In, addition, MALDI-TOF showed that uncharacterized post-translational modifications had occurred particularly in (A).

Gel formation was qualitatively assessed for the (A), (B), and (C) triblock gelatins. To this end, solutions of the three purified proteins in water at 10% (w/v) were prepared and heated for 5 min. at 45 °C to melt any triple helices. The gels were then allowed to cool to room temperature. Gel formation within 5 min. was observed only for (C), as judged by the conventional "inverted tube test". After overnight incubation all triblocks formed gels.

Example 3

Apolypeptide (D) was made, as a nonablock variant of (C) of Example 1, i.e. with the integer n being 4. A corresponding gene was constructed, encoding a 915 aa (83 kDa) protein with a calculated isoelectric point of 4.63. (D)

- 915 amino acids
- molecular weight: 82.64 kDa
- IEP: 4.63
- Amino acid sequence:

**(GPP)$_9$**-GPKGEPGSPGENGPKGNSGEPGPSGEPGKQGPSGSPGEQGPKG
EPGSPGENGPKGNSGEPGPSGEPGKQGPSGSPGEQGPKGEPGSPGENG
PKGNSGEPGPSGEPGKQGPSGSPGEQGPKGEPGSPGENGPKGNSGEPG
PSGEPGKQGPSGSPGEQGPKGEPGSPGENGPKGNSGEPGPSGEPGKQG
PSGSPGEQ-**(GPP)$_9$**-GPKGEPGSPGENGPKGNSGEPGPSGEPGKQGPSGS
PGEQGPKGEPGSPGENGPKGNSGEPGPSGEPGKQGPSGSPGEQGPKGE
PGSPGENGPKGNSGEPGPSGEPGKQGPSGSPGEQGPKGEPGSPGENGP

KGNSGEPGPSGEPGKQGPSGSPGEQGPKGEPGSPGENGPKGNSGEPGP

SGEPGKQGPSGSPGEQ-**(GPP)$_9$**-GPKGEPGSPGENGPKGNSGEPGPSGEP

GKQGPSGSPGEQGPKGEPGSPGENGPKGNSGEPGPSGEPGKQGPSGSP

GEQGPKGEPGSPGENGPKGNSGEPGPSGEPGKQGPSGSPGEQGPKGEP

GSPGENGPKGNSGEPGPSGEPGKQGPSGSPGEQGPKGEPGSPGENGPK

GNSGEPGPSGEPGKQGPSGSPGEQ-**(GPP)$_9$**-

GPKGEPGSPGENGPKGNSGEPGPSGEPGKQGPSGSPGEQGPKGEPGSP

GENGPKGNSGEPGPSGEPGKQGPSGSPGEQGPKGEPGSPGENGPKGNS

GEPGPSGEPGKQGPSGSPGEQGPKGEPGSPGENGPKGNSGEPGPSGEP

GKQGPSGSPGEQGPKGEPGSPGENGPKGNSGEPGPSGEPGKQGPSGSP

GEQ-**(GPP)$_9$**

The cloning procedure used to generate the nonablock constructs is shown in Figure 1. The sequence of the construct was verified by Sanger sequencing from both ends, and by Nanopore long read sequencing to exclude the hypothetical possibility of rearrangements in the middle section (this cannot normally be verified by Sanger sequencing because of the long repetitive sequence). The vector was then used to transform *P. pastoris.*

The nonablock gelatin (D) was successfully produced.

**Claims**

1.  A polypeptide having an amino acid sequence comprising the oligopeptides of formula (II), (III), (IV), and (V):

    GPKGEPGSPGEN          (II)

    GPKGNSGEP          (III)

    GPSGEPGKQGPS          (IV)

    GSPGEQ          (V).

2.  The polypeptide of claim 1, having the amino acid sequence (GPKGEPGSPGENGPKGNSGEPGPSGEPGKQGPSGSPGEQ)$_q$ wherein q is an integer of from 1 to 40, preferably 3 to 10.

3.  A block co-polypeptide having a structure $(T_{block}$-$G_{block})_n$-$T_{block}$, wherein n is an integer of from 1 to 18; $T_{block}$ denoted, each independently, a trimerizing peptide block having an amino acid sequence selected from the group consisting of $(GPP)_k$, $(GER)_m$, $(GRE)_m$, $(GEK)_m$, $(GKE)_m$, $(GPKGDP)_p$, and $(GPKGEP)_p$, wherein k is an integer from 6 to 20, preferably 7 to 16, more preferably 8 to 12, m is an integer of from 12 to 20 and p is an integer of from 3 to 12; and $G_{block}$ represents a polypeptide as defined in claim 1 or 2.

4.  The block co-polypeptide of claim 3, wherein the $T_{block}$ modules are $(GPP)_k$, and k is 8 to 12.

5.  A hydrogel comprising a block co-polypeptide as defined in claim 3 or 4.

6.  A nucleic acid construct encoding the block co-polypeptide as defined in claim 3 or 4.

7.  An expression vector comprising the nucleic acid construct of claim 6.

8.  The expression vector of claim 7, wherein the vector is a *Pichia pastoris* expression vector.

**9.** A host cell comprising the expression vector of claim 7.

**10.** The host cell of claim 9, being a *Pichia pastoris* host cell comprising the expression vector of claim 8.

**11.** A method of preparing a polypeptide, comprising:

a) introducing the expression vector of claim 7 into host cells;
b) culturing the host cells in a culture medium, under conditions allowing the expression of the polypeptide in said host cells and secretion of the polypeptide into said culture medium;
c) removing said host cells from the culture medium, so as to provide a cell-free culture medium containing the secreted polypeptide by microfiltration, optionally preceded by centrifugation;
d) optionally concentrating the polypeptide in the cell-free culture medium by ultrafiltration
e) optionally removing low molecular weight components from the polypeptide and culture medium by ultrafiltration/diafiltration
f) precipitating specifically the polypeptide from the cell-free culture medium by differential precipitation with a salt, preferably with ammonium sulfate, preferably at 20-80% of the saturating ammonium sulfate concentration, more preferably at 40-60% of the saturating ammonium sulfate concentration;
g) dissolving the precipitated polypeptide in water;
h) optionally repeating the above precipitation and dissolution of the polypeptide;
i) removing the salt from the polypeptide and optionally concentrating the polypeptide by ultrafiltration/diafiltration or dialysis,
j) optionally drying the polypeptide by spray drying or freeze-drying.

**12.** A method of preparing a polypeptide, comprising:

a) introducing the expression vector of claim 7 into host cells;
b) culturing the host cells in a culture medium, under conditions allowing the expression of the polypeptide in said host cells and secretion of the polypeptide into the extracellular culture medium;
c) removing said host cells from the culture medium containing the secreted polypeptide by microfiltration, optionally preceded by centrifugation;
d) optionally concentrating the polypeptide in the cell-free culture medium by ultrafiltration
e) optionally removing low molecular weight components from the polypeptide and culture medium by ultrafiltration/diafiltration
f) purifying the polypeptide by chromatography, for example anion exchange chromatography
g) removing salts and/or buffers from the polypeptide and optionally concentrating the polypeptide by ultrafiltration/diafiltration or dialysis,
h) optionally drying the polypeptide by spray drying or freeze-drying.

**13.** A method of preparing a polypeptide, comprising:

a) introducing the expression vector of claim 7 into host cells;
b) culturing the host cells in a culture medium, under conditions allowing the expression of the polypeptide in said host cells;
c) lysing the host cells resulting in a dispersion comprising the polypeptide and lysed cell debris;
d) separating said debris from the dispersion, by a separation technique selected from the group consisting of centrifugation,, ultrafiltration, microfiltration, and combinations thereof;
e) optionally removing low-molecular weight components from the polypeptide-containing dispersion by ultrafiltration or dialysis;
f) purifying the polypeptide by a combination of differential precipitation and or chromatography;
g) desalting the polypeptide obtained in step (d), (e) or (f) by ultrafiltration or dialysis and optionally drying the polypeptide.

**14.** The method of claim 11, 12 or13, wherein the expression vector is as defined in claim 8, and wherein the host cells are *Pichia pastoris* host cells.

**15.** Use of a block-co-polypeptide according to claim 3 or 4 as an ingredient capable of gelling in food and beverage applications, particularly selected from the group consisting of desserts, fruit gummies, marshmallows, pastilles caramels yogurt, meat, sausages, broths, and canned meats.

16. Use of a block-co-polypeptide according to claim 3 or 4 as a rheology modifier in cosmetics or personal care formulations.

17. Use of a block-co-polypeptide according to claim 3 or 4 as a skin-restoring agent in cosmetics or personal care formulations.

18. Use of a block-co-polypeptide according to claim 3 or 4 for the production of capsules for pharmaceutical formulations.

19. Use of a block-co-polypeptide according to claim 3 or 4 for the production of gels for the controlled and/or extended release of substances.

20. Use of a block-co-polypeptide according to claim 3 or 4 as a scaffold material for tissue culture or tissue engineering.

21. Use of a block-co-polypeptide according to claim 3 or 4, as an additive to promote the stability, viability and/or shelf life of cells, tissues, or vaccines.

22. Use of a block-co-polypeptide according to claim 3 or 4, either in gel or in dry form, as a surgical aid to prevent post-operative sticking of tissues or organs to each other.

23. Use of a block-co-polypeptide according to claim 3 or 4, either in gel or in dry form as an aid in surgery to control blood flow.

Fig. 1

Europäisches
Patentamt

European
Patent Office

Office européen
des brevets

# EUROPEAN SEARCH REPORT

Application Number

**EP 23 16 7140**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2009/151327 A1 (STICHTING DIENST LANDBOUWKUNDI [NL] ET AL.) 17 December 2009 (2009-12-17) | 1,3-15 | INV. C07K14/00 C07K14/78 C12N15/81 C12P21/02 |
| A | * claims 1-24 * | 2 | |
| X | WO 2010/079076 A2 (MOSAIQUES DIAGNOSTICS & THERAP [DE]; MISCHAK HARALD [DE]) 15 July 2010 (2010-07-15) | 1,3-15 | ADD. C12R1/84 |
| A | * claim 12 * * sequence 75 * | 2 | |
| X | WO 2010/091251 A2 (UNIV NEW JERSEY MEDICINE&DENTISTRY) 12 August 2010 (2010-08-12) | 1,3-15 | |
| A | * paragraph [0010] * * sequence 45 * | 2 | |
| X | CN 114 874 316 A (UNIV CHONGQING) 9 August 2022 (2022-08-09) | 1,3-15 | |
| A | * sequence 1 * | 2 | |
| X | EP 3 315 145 A1 (BSN MEDICAL GMBH [DE]) 2 May 2018 (2018-05-02) | 1,3-15 | |
| A | * sequence 5 * | 2 | |
| A | WERTEN M W T ET AL: "Secreted production of a custom-designed, highly hydrophilic gelatin in Pichia pastoris", PROTEIN ENGINEERING, OXFORD UNIVERSITY PRESS, SURREY, GB, vol. 14, no. 6, 1 June 2001 (2001-06-01), pages 447-454, XP002302730, ISSN: 0269-2139, DOI: 10.1093/PROTEIN/14.6.447 * abstract * * page 447 - page 454 * | 1-15 | |

TECHNICAL FIELDS
SEARCHED (IPC)

C07K
C40B
C12P
C12N
C12R

The present search report has been drawn up for all claims

| Place of search | Date of completion of the search | Examiner |
|---|---|---|
| Munich | 23 November 2023 | Tudor, Mark |

EPO FORM 1503 03.82 (P04C01)

## ANNEX TO THE EUROPEAN SEARCH REPORT
## ON EUROPEAN PATENT APPLICATION NO.

EP 23 16 7140

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2023

| Patent document cited in search report | | Publication date | Patent family member(s) | | Publication date |
|---|---|---|---|---|---|
| WO 2009151327 | A1 | 17-12-2009 | EP | 2133364 A1 | 16-12-2009 |
| | | | WO | 2009151327 A1 | 17-12-2009 |
| WO 2010079076 | A2 | 15-07-2010 | EP | 2394171 A2 | 14-12-2011 |
| | | | US | 2011297543 A1 | 08-12-2011 |
| | | | US | 2015346150 A1 | 03-12-2015 |
| | | | WO | 2010079076 A2 | 15-07-2010 |
| WO 2010091251 | A2 | 12-08-2010 | US | 2012116053 A1 | 10-05-2012 |
| | | | US | 2017044220 A1 | 16-02-2017 |
| | | | WO | 2010091251 A2 | 12-08-2010 |
| CN 114874316 | A | 09-08-2022 | NONE | | |
| EP 3315145 | A1 | 02-05-2018 | EP | 3315145 A1 | 02-05-2018 |
| | | | WO | 2018078084 A1 | 03-05-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009151327 A

### Non-patent literature cited in the description

- **WERTEN et al.** *Biomacromolecules,* 2009, vol. 10, 1106-1113
- **WERTEN et al.** *Protein Engineering,* 2001, vol. 14 (6), 447-454
- **WERTEN et al.** *Yeast,* vol. 15 (11), 1087-1096
- **PADGETT K.A. ; SORGE J.A.** *Gene,* 1996, vol. 168, 31-35
- **MCMILLAN R.A. ; LEE T.A.T. ; CONTICELLO V.P.** *Macromolecules,* 1999, vol. 32, 3643-3648
- **WERTEN M.W.T. ; WISSELINK W.H. ; JANSEN-VAN DEN BOSCH T.J. ; DE BRUIN E.C. ; DE WOLF F.A.** *Protein Engineering,* 2001, vol. 14 (6), 447-454
- **GOEDEN-WOOD N.L. ; CONTICELLO V.P. ; MULLER S.J. ; KEASLING J.D.** *Biomacromolecules,* 2002, vol. 3, 874-879
- **WON J.-I. ; BARRON A.E.** *Macromolecules,* 2002, vol. 35, 8281-8287
- **HENDERSON D.B. ; DAVIS R.M. ; DUCKER W.A. ; VAN COTT K.E.** *Biomacromolecules,* 2005, vol. 6, 1912-1920
- **LU Q.** *Trends in Biotechnol.,* 2005, vol. 23 (4), 199-207
- **MI L.** *Biomacromolecules,* 2006, vol. 7, 2099-2107